# EUROPEAN PATENT SPECIFICATION

(11) **EP 3 792 341 B1**
(45) Date of publication and mention of the grant of the patent: **06.11.2024**
(21) Application number: 19382798.7
(22) Date of filing: 16.09.2019
(51) Int. Cl.: C12M 3/00, A61F 2/28, A61F 2/30, C12M 1/12, C12M 1/26, C12M 1/42

(54) **METHOD FOR GENERATING A TISSUE**
VERFAHREN ZUR HERSTELLUNG EINES GEWEBES
PROCÉDÉ DE GÉNÉRATION D'UN TISSU

(43) Date of publication of application: 17.03.2021
(73) Proprietor: Regemat 3D S.L., 18016 Granada (ES)
(72) Inventor: BAENA MARTÍNEZ, José Manuel, E-18016 Granada (ES)
(74) Representative: TRBL Intellectual Property

(56) References cited:
- EP-A1- 3 103 415
- CN-A- 105 013 011
- US-A1- 2004 219 659
- US-A1- 2019 105 429
- US-B2- 7 410 792

## Description

### TECHNICAL FIELD

This invention is related to the field of those apparatuses and methods for generating tissue-engineered products, and more particularly, to the field of those comprising bioreactors.

### STATE OF THE ART

Much effort has been aimed to the creation of functional tissues and organs in laboratory. The lack of tissue regeneration in human beings and the deficiency of allogenic transplants in addition to the increasing of life expectancy make this problem to be considered as one of the most important ones of humanity in the current era.

A bioreactor is an engineered device that supports a biologically active environment. Bioreactors play a crucial role in the final properties of engineered tissues.

There are many examples of bioreactors configured to generate tissue elements from a cell culture. A scaffold is commonly used as a support for cell growing, provided the suitable environment conditions inside the bioreactor. The scaffold may be subject to some mechanic loads, that activate the biological behaviour of the cells, generating the substances needed to mimic the histology of a functional living tissue and to improve the mechanical properties of the resulting engineered tissue.

Document US 2019/105429 A1 discloses a hybrid bone-implant graft comprising an implant material and an engineered bone tissue graft having a scaffold, wherein the graft is composed of cells seeded on the scaffold and cultured to promote attachment of the cells to the implant material. Document EP 3103415 A1 discloses a method of making a tissue structure, the method comprising forming an image of a target tissue structure, shaping a three-dimensional scaffold responsively to the image, seeding the scaffold with cells and delivering nutrients to the cells within and on the surface of the scaffold by flowing a nutrient fluid into a tightly conforming vessel holding the scaffold, through multiple first surface portions of the scaffold, and out through at least one second surface portion. However, none of these documents is related to the specific problems of human joints.

The bioreactor environment directly affects the uniformity of cell seeding into 3D scaffolds as well as the maintenance of the cell phenotype and characteristics of the tissue. A bioreactor should provide *in* vivo-like physical stimulation to the growing tissues to promote secretion of ECM and tissue formation.

However, this generating process is not always successful when generating joints tissues amongst others, since it is difficult to choose which loads are better simulating the final loads that the tissue will face when installed in the human body.

An alternative solution for this problem is therefore sought.

### SUMMARY OF THE INVENTION

The invention provides an alternative solution for generating joint tissue engineered products by a method for generating a tissue according to claim 1.

Preferred embodiments of the invention are defined in dependent claims.

Unless otherwise defined, all terms (including technical and scientific terms) used herein are to be interpreted as is customary in the art. It will be further understood that terms in common usage should also be interpreted as is customary in the relevant art and not in an idealised or overly formal sense unless expressly so defined herein.

In this text, the term "comprises" and its derivations (such as "comprising", etc.) should not be understood in an excluding sense, that is, these terms should not be interpreted as excluding the possibility that what is described and defined may include further elements, steps, etc.

In a first inventive aspect, the invention provides a method for generating a tissue, the method comprising the steps of
providing a physical model of a patient's joint;
identify a damaged element which is intended to be replaced by a generated tissue;
replacing the damaged element of the physical model with a scaffold;
introducing the physical model with the scaffold in a bioreactor and start cell culture around the scaffold;
make the physical model simulate standard joint movements while the cell culture is taking place, so that the tissue is generated around the scaffold.

With this method, the tissue is generated with a load pattern which is equal to the loads that the tissue will have to bear when finally installed in the patient's real joint, due to the fact that, in this case, the loads are not predetermined loads performed by an external machine, but the loads that the joint physical model will cause on the scaffold when simulating standard movements of the real joint.

In this context, the expression "around the scaffold" makes reference to any location close to any point of the scaffold, either in its exterior or interior periphery, since the cell culture takes place in all these places.

In some particular embodiments, the tissue is one of cartilage tissue, osteochondral tissue, meniscus tissue or bone tissue.

These are some examples of tissues that may be generated by a method according to the invention. In each of these different cases, the type of cell culture and the environment conditions are modified to match the creation of the corresponding tissue.

In some particular embodiments, the physical model of the patient's joint has been generated from a scanned image of the patient's real joint. In particular embodiments, the patient's real joint is previously scanned by computed tomography or by magnetic resonance imaging.

In these cases, the joint physical model has not been selected from a predetermined database, but it is obtained as a copy of the patient's real joint, thus increasing the similarity between the real loads caused in the patient's joint and the loads applied during the tissue generation process.

In some particular embodiments, in the step of replacing the damaged element, the scaffold contains cells and/or biomolecules.

In these cases, the loads applied on the scaffold will affect the cells from the beginning of the process.

In some particular embodiments, at least one of the following conditions is satisfied
the CO2 concentration is kept between 0 and 20 % during the cell culture;
the pH is kept between 6,8 and 8 during the cell culture;
the temperature is kept between 4 and 41 degrees Celsius during the cell culture; or;
the humidity is kept between 90 and 100 % relative humidity during the cell culture. These ranges provide values which are suitable in the generation of different tissues that may be created by the method according to the invention. The pH is kept in these values to model pathological conditions as gout, and the range of temperatures covers both the conditions at standard cell culture and the extreme physiological conditions.

In some particular embodiments, the physical model comprises one of a knee joint, a hip joint, a mandible, an elbow joint, an ankle joint, a wrist joint, a shoulder joint, a temporomandibular joint, a trachea, a vertebrae joint, a carpometacarpal joint or interphalangeal joints.

These are some particular examples of joints where tissue may be generated with a method according to the invention.

In some particular embodiments, the step of simulating standard movements is carried out by gripping two or more points of the physical model and moving them. In particular embodiments, the standard movements comprise relative displacement between the two points, compression, traction and rotation.

By the simulation of these movements, the loads generated in the cells inside the scaffold are under the same loads that if they were located in the final position in the patient's real joint.

### BRIEF LIST OF DRAWINGS AND REFERENCE NUMBERS

To complete the description and in order to provide for a better understanding of the invention, a set of drawings is provided. Said drawings form an integral part of the description and illustrate an embodiment of the invention, which should not be interpreted as restricting the scope of the invention, but just as an example of how the invention can be carried out. The drawings comprise the following figures:
Figure 1 shows a first step of a particular embodiment of a method of generating a tissue according to the invention, where a patient is located in a magnetic resonance machine.
Figures 2a and 2b show some further steps of this method.
Figure 3 shows a bioreactor suitable for carrying out a method according to the invention.

Elements of the example embodiments are consistently denoted by the same reference numerals throughout the drawings and detailed description where appropriate:
- 1: Physical model of a patient's knee
- 2: Damaged element
- 3: Scaffold
- 4: Patient's real knee
- 10: Bioreactor
- 11: Chamber
- 12: Sensors
- 13: Actuators
- 14: Mechanical actuators
- 15: Fluid recirculation system

### DETAILED DESCRIPTION OF THE INVENTION

The example embodiments are described in sufficient detail to enable those of ordinary skill in the art to embody and implement the systems and processes herein described. It is important to understand that embodiments can be provided in many alternate forms and should not be construed as limited to the examples set forth herein.

Accordingly, while embodiment can be modified in various ways and take on various alternative forms, specific embodiments thereof are shown in the drawings and described in detail below as examples. There is no intent to limit to the particular forms disclosed. On the contrary, all modifications, equivalents, and alternatives falling within the scope of the appended claims should be included.

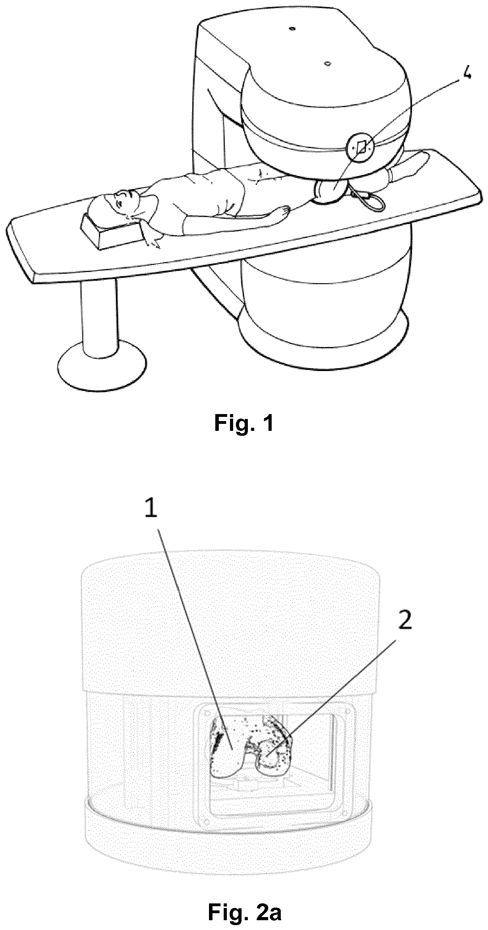

Figure 1 shows a first step of a particular embodiment of a method of generating a tissue according to the invention. A patient has an injury in their knee, and needs a cartilage generated by a method according to the invention. In this first step, magnetic resonance image is used to create a scanned image of the patient's real knee 4.

This scanned image is used to create a physical model of the patient's knee cartilage, so that this physical model may be used in further steps of the method.

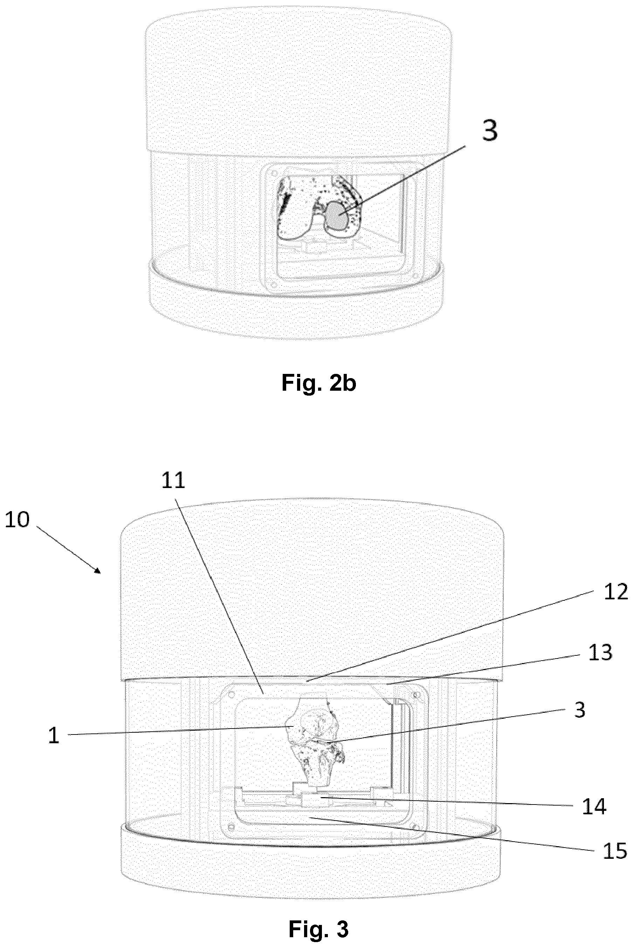

Figures 2a and 2b show some further steps of this method. In figure 2a, the knee physical model 1 is created by additive manufacturing technologies as 3D printing, and then a damaged element 2 is identified in this knee physical model 1. In different embodiments, this damaged element 2 may be a cartilage, a meniscus or some osteochondral tissue. The method of the invention is valid for a wide range of tissues.

Figure 2b shows how the damaged element 2 is replaced by a scaffold 3, which will be used in a further step of the method. The scaffold 3 is located in the same position as the damaged element was, to be subject to the same loads and environmental conditions as the damaged element was. The scaffold could be manufactured using bioprinting technologies or other available manufacturing procedure, depending on the biomaterials used. The internal geometry of the scaffold will be relevant to promote the tissue formation in the damaged element as play a main role in the loads transferred to the cells, the biological behaviour of them and the extracellular matrix created.

Figure 3 shows a bioreactor 10 suitable for carrying out a method according to the invention. This bioreactor 10 comprises
a chamber 11;
some sensors 12 configured to measure at least one of CO2 concentration, pH, temperature or humidity;
some actuators 13 to control de range of at least one of CO2 concentration, pH, temperature or humidity;
mechanical actuators14 configured to move the knee physical model 1; and
a fluid recirculating system 15.

The knee physical model 1 is introduced in this bioreactor 10, and a cell culture takes place around the scaffold 3, to generate the tissue that will be later on placed on the patient's real knee.

To improve the mechanical properties of the generated tissue, some loads are applied on the scaffold 3 during cell culture. The novelty of this method is that these loads are not a set of predetermined loads which have been extracted from a database, but are the loads that the real knee will cause on the generated tissue, with the stress distribution caused by the real surface shape of the patients' knee so the result is far more realistic than in the methods known in the state of the art.

To apply these loads, some standard movements are generated in the knee physical model 1. Two points of the knee physical model 1 are grabbed by the mechanical actuators 14 and then some movements, such as relative displacement, compression, traction and rotation, are being performed. The performance of these actions during the cell culture achieve a better result in the mechanical properties of the tissue.

Environmental parameters are also controlled by the bioreactor. In particular, for this type of tissue, the following conditions are satisfied
the CO2 concentration is kept between 0 and 20 % during the cell culture;
the pH is kept between 6,8 and 8 during the cell culture (to model pathological conditions as gout); or
the temperature is kept between 4 and 41 degrees Celsius during the cell culture to mimic the condition given in a standard cell culture or at higher temperatures (cold storage and extreme physiological conditions);
the humidity is kept between 90 and 100 % relative humidity during the cell culture.

## Claims

1. Method for generating a tissue, the method comprising the steps of
providing a physical model of a patient's joint (1);
identify a damaged element (2) which is intended to be replaced by a generated tissue;
replacing the damaged element (2) of the physical model with a scaffold (3);
introducing the physical model (1) with the scaffold (3) in a bioreactor (10) and start cell culture around the scaffold (3);
make the physical model (1) simulate standard joint movements while the cell culture is taking place, so that the tissue is generated around the scaffold (3).

2. Method according to claim 1, wherein the tissue is one of cartilage tissue, osteochondral tissue, meniscus tissue or bone tissue.

3. Method according to any of the preceding claims, wherein the physical model (1) of the patient's joint has been generated from a scanned image of the patient's real joint (4).

4. Method according to claim 3, further comprising the step of scanning the patient's real joint (4) by computed tomography of by magnetic resonance imaging.

5. Method according to any of the preceding claims, wherein in the step of replacing the damaged element, the scaffold contains cells and/or biomolecules.

6. Method according to any of the preceding claims, wherein at least one of the following conditions is satisfied
the CO2 concentration is kept between 0 and 20 % during the cell culture;
the pH is kept between 6,8 and 8 during the cell culture;
the temperature is kept between 4 and 41 degrees Celsius during the cell culture; or
the humidity is kept between 90 and 100 % relative humidity during the cell culture.

7. Method according to any of the preceding claims, wherein the physical model comprises one of a knee joint, a hip joint, a mandible, an elbow joint, an ankle joint, a wrist joint, a shoulder joint, a temporomandibular joint, a trachea, a vertebrae joint, a carpometacarpal joint or interphalangeal joints.

8. Method according to any of the preceding claims, wherein the step of simulating standard movements is carried out by gripping two or more points of the physical model(1) and moving them.

9. Method according to claim 8, wherein the standard movements comprise relative displacement between the two points, compression, traction and rotation.

## Patentansprüche

1. Verfahren zur Herstellung eines Gewebes, wobei das Verfahren folgende Schritte umfasst:
Bereitstellen eines physischen Modells des Patientengelenks (1);
Identifizieren eines beschädigten Elements (2), das dazu bestimmt ist, durch ein hergestelltes Gewebe ersetzt zu werden;
Ersetzen des beschädigten Elements (2) des physischen Modells durch ein Gerüst (3);
Einführen des physischen Modells (1) mit dem Gerüst (3) in einen Bioreaktor (10) und Starten der Zellkultur um das Gerüst (3) herum;
Veranlassen, dass das physische Modell (1) standardmäßige Gelenkbewegungen simuliert, während die Zellkultur stattfindet, so dass das Gewebe um das Gerüst (3) herum hergestellt wird.

2. Verfahren nach Anspruch 1, wobei das Gewebe eines von Knorpelgewebe, osteochondrales Gewebe, Meniskusgewebe oder Knochengewebe ist.

3. Verfahren nach einem der vorhergehenden Ansprüche, wobei das physische Modell (1) des Patientengelenks aus einem gescannten Bild des echten Gelenks (4) des Patienten hergestellt wurde.

4. Verfahren nach Anspruch 3, ferner umfassend den Schritt des Scannens des echten Gelenks (4) des Patienten durch Computertomographie oder Magnetresonanzbildgebung.

5. Verfahren nach einem der vorhergehenden Ansprüche, wobei in dem Schritt des Ersetzens des beschädigten Elements das Gerüst Zellen und/oder Biomoleküle enthält.

6. Verfahren nach einem der vorhergehenden Ansprüche, wobei mindestens eine der folgenden Bedingungen erfüllt ist:
der CO2-Gehalt wird während der Zellkultur zwischen 0 und 20 % gehalten;
der pH-Wert wird während der Zellkultur zwischen 6,8 und 8 gehalten;
die Temperatur wird während der Zellkultur zwischen 4 und 41 Grad Celsius gehalten; oder
die Feuchtigkeit wird während der Zellkultur zwischen 90 und 100 % relativer Feuchtigkeit gehalten.

7. Verfahren nach einem der vorhergehenden Ansprüche, wobei das physische Modell eines von Kniegelenk, Hüftgelenk, Unterkiefer, Ellbogengelenk, Fußgelenk, Handgelenk, Schultergelenk, Kiefergelenk, Trachea, Wirbelgelenk, Karpometakarpalgelenk oder Interphalangealgelenk umfasst.

8. Verfahren nach einem der vorhergehenden Ansprüche, wobei der Schritt des Simulierens von standardmäßigen Bewegungen durchgeführt wird, indem zwei oder mehr Punkte des physischen Modells (1) ergriffen und bewegt werden.

9. Verfahren nach Anspruch 8, wobei die standardmäßigen Bewegungen eine relative Verschiebung zwischen den beiden Punkten, Kompression, Zug und Rotation umfassen.

## Revendications

1. Procédé de génération d'un tissu, le procédé comprenant les étapes de
fournir un modèle physique d'une articulation d'un patient (1) ;
identifier un élément endommagé (2) destiné à être remplacé par un tissu généré ;
remplacer l'élément endommagé (2) du modèle physique par un échafaudage (3) ;
introduire le modèle physique (1) avec l'échafaudage (3) dans un bioréacteur (10) et commencer une culture cellulaire autour de l'échafaudage (3) ;
faire en sorte que le modèle physique (1) simule des mouvements articulaires standard pendant que la culture cellulaire a lieu, de façon à ce que le tissu soit généré autour de l'échafaudage (3).

2. Procédé selon la revendication 1, dans lequel le tissu est l'un d'un tissu cartilagineux, un tissu ostéochondral, un tissu méniscal ou un tissu osseux.

3. Procédé selon l'une quelconque des revendications précédentes, dans lequel le modèle physique (1) de l'articulation du patient a été généré à partir d'une image scannée de l'articulation réelle du patient (4).

4. Procédé selon la revendication 3, comprenant en outre l'étape consistant à scanner l'articulation réelle du patient (4) par tomographie assistée par ordinateur ou par imagerie par résonance magnétique.

5. Procédé selon l'une quelconque des revendications précédentes, dans lequel, lors de l'étape de remplacement de l'élément endommagé, l'échafaudage contient des cellules et/ou des biomolécules.

6. Procédé selon l'une quelconque des revendications précédentes, dans lequel au moins l'une des conditions suivantes est remplie
la concentration en CO₂ est maintenue entre 0 et 20 % pendant la culture cellulaire ;
le pH est maintenu entre 6,8 et 8 pendant la culture cellulaire ;
la température est maintenue entre 4 et 41 degrés Celsius pendant la culture cellulaire ; ou
l'humidité est maintenue entre 90 et 100 % d'humidité relative pendant la culture cellulaire.

7. Procédé selon l'une quelconque des revendications précédentes, dans lequel le modèle physique comprend l'une d'une articulation du genou, une articulation de la hanche, une mandibule, une articulation du coude, une articulation de la cheville, une articulation du poignet, une articulation de l'épaule, une articulation temporo-mandibulaire, une trachée, une articulation des vertèbres, une articulation carpométacarpienne ou des articulations interphalangiennes.

8. Procédé selon l'une quelconque des revendications précédentes, dans lequel l'étape de simulation des mouvements standards est réalisée en saisissant deux ou plusieurs points du modèle physique(1) et en les déplaçant.

9. Procédé selon la revendication 8, dans lequel les mouvements standards comprennent le déplacement relatif entre les deux points, la compression, la traction et la rotation.
